# EUROPÄISCHE PATENTSCHRIFT

(11) **EP 1 432 685 B1**
(45) Veröffentlichungstag und Bekanntmachung des Hinweises auf die Patenterteilung: **09.03.2005**
(21) Anmeldenummer: 02777103.9
(22) Anmeldetag: 14.09.2002
(51) Int. Cl.: C07D 213/82, C07C 233/64, A01N 43/40

(54) **KRISTALLINE HYDRATE VON ANILID-DERIVATEN ALS FUNIGIZIDE UND INSEKTIZIDE**
CRYSTALLINE HYDRATES OF ANILID DERIVATIVES AS FUNGICIDES AND INSECTICIDES
HYDRATES CRISTALLINS DE DERIVES D'ANILIDE COMME FUNGICIDES ET INSECTICIDES

(30) Priorität: 25.09.2001 DE 10147034
(43) Veröffentlichungstag der Anmeldung: 30.06.2004
(73) Patentinhaber: BASF AKTIENGESELLSCHAFT, 67056 Ludwigshafen (DE)
(72) Erfinder: BRATZ, Matthias, Raleigh, NC 27613 (US); WIGGER, August, 95478 Kemnath-Stadt (DE); ERK, Peter, 67227 Frankenthal (DE); ZIEGLER, Hans, 67112 Mutterstadt (DE); KRÖHL, Thomas, 55129 Mainz (DE); JÄGER, Karl-Friedrich, 67117 Limburgerhof (DE); MAYER, Horst, CEP-12513-300 Guaratingueta (BR)
(86) Internationale Anmeldenummer: PCT/EP2002/010320
(87) Internationale Veröffentlichungsnummer: WO 2003/029219

(56) Entgegenhaltungen:
- WO-A-95/15690
- WO-A-99/31979

## Beschreibung

Gegenstand der vorliegenden Erfindung sind kristalline substituierte nilid-Derivate der Formel I 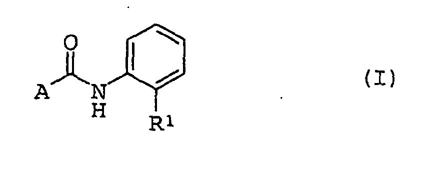 worin
- A: für 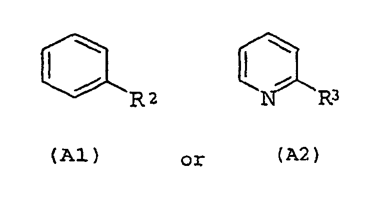 steht,
- R¹: für Phenyl steht, das durch Halogen substituiert ist,
- R²: für Methyl, Difluormethyl, Trifluormethyl, Chlor, Brom oder Jod steht,
- R³: für Trifluormethyl oder Chlor steht,
dadurch gekennzeichnet, dass sie als Hydrate vorliegen. Die vorliegende Erfindung umfasst weiterhin die Herstellung von Suspensionskonzentraten sowie Suspoemulsionen unter Verwendung des oben genannten Wirkstoffhydrates sowie Verfahren zur Bekämpfung von phytopathogenen Pilzen, unerwünschtem Insekten- oder Milbenbefall und/oder zur Regulation des Wachstums von Pflanzen durch Verdünnen der oben genannten Formulierungen.

Phenylamide ähnlich denen der Formel I (A=A1) und deren Verwendung als Fungizide sind bekannt (WO 9515690). Zu dem sind die fungizide Wirkung von Pyridylamiden der Formel I (A=A2), eine Klasse von im Wesentlichen wasserunlöslichen Verbindungen, sowie Verfahren zu deren Herstellung bekannt (EP-A2 545099, WO 99 31 979). Technisch erhältich sind diese Verbindungen durch Kristallisation aus einem organischen Lösungsmittel.

Will man wasserunlösliche Verbindungen zwecks Applikation auf die entsprechenden Schädlinge oder Pflanzen in flüssiger Form formulieren, gibt es prinzipiell zwei Möglichkeiten:
1. Der Wirkstoff wird als Emulsionskonzentrat (EC) formuliert.
2. Der Wirkstoff wird als wässriges Suspensionskonzentrat (SC) formuliert. Hierbei kann dem SC noch eine organische Phase hinzugefügt werden, die optional eine weitere Hilfstoffe oder Wirkstoffe enthalten kann. Die hergestellte Formulierung bezeichnet man als Suspoemulsion (SE).

Die Bereitstellung wässriger Formulierungen ist unter Berücksichtigung der Umweltbelastung durch organische Lösungsmittel, wie sie in großen Mengen in ECs verwendet werden, vorteilhaft.

SCs bestehen im wesentlichen aus einer wässrigen Phase, in welcher der Wirkstoff nebst Hilfstoffen suspendiert ist. Die Herstellung von SCs ist dem Fachmann bekannt. Sie kann z.Bsp. durch Vermahlen des oben genannten Wirkstoffes in Gegenwart diverser Hilfsstoffe sowie Wasser als kontinuierlichem Medium erfolgen (Mollet, H. und Grubemann, A. "Formulierungstechnik", WILEY-VCH, 2000, S. 133 ff.).

Unter dem Begriff Hilfstoffe sind hier Formulierungshilfsmittel wie Tenside, Andicker , Lösungsmittel, Antischaummittel, Bakterizide und Frostschutzmittel zu verstehen.

Genannte SC' s können zur Herstellung von SE' s verwendet werden. Dies geschieht in der Regel dann, wenn der bzw. die in der Suspension enthaltene Wirkstoffe mit flüssigen, nicht wassermischbaren Wirkstoffen, Wirkstoffölen oder mit einer organischen Wirkstofflösung eines nicht wasserlöslichen Wirkstoffes in einer Fertigformulierung kombiniert werden sollen. Die Herstellung von SEs ist dem Fachmann bekannt und kann z.Bsp. nach dem in EP-A 707445 beschriebenen Verfahren erfolgen.

Bei der Herstellung der oben erwähnten SCs ist es erforderlich, den Wirkstoff in Gegenwart von Wasser und weiterer Hilfstoffe möglichst fein zu vermahlen.

Dies ist jedoch bei den oben genannten Nicotinsäureanilid-Derivaten überraschender Weise nicht möglich, da diese bei der Herstellung eines SCs einen lehmartigen Feststoff bilden, welcher einen weiteren Mahlvorgang verhindert.

Aufgabe der vorliegenden Erfindung war somit, den Wirkstoff dergestalt zu modifizieren, dass eine Vermahlung mit Hilfstoffen in Gegenwart von Wasser erfolgen kann.

Die Aufgabe wurde gelöst durch die Bereitstellung der entsprechenden Hydrate von Anilid-Derivaten. Überaschender Weise wurde gefunden, dass die Vermahlung der Hydrate im Gegensatz zu den Anhydraten ohne Probleme erfolgen konnte. Gegenstand der vorliegenden Erfindung sind die Anilidderivat(Hydrate der Formel I: 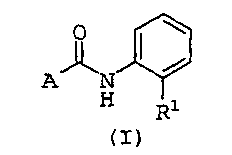 worin
- A: für 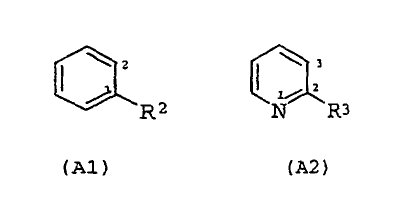
- R¹: für Phenyl steht, das durch Halogen substituiert ist;
- R²: für Methyl, Difluormethyl, Trifluormethyl, Chlor, Brom oder Jod steht,
- R³: für Trifluormethyl oder Halogen steht: wobei die Verknüpfung von A1 in 2-Postion und von A2 in 3 Position zur Stanmverbindung I bevorzugt ist.

Hierbei sind Hydrate von Nicotinsäureanilid-Derivaten der Formel II besonders bevorzugt: 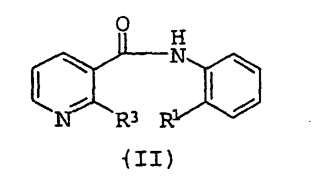 worin
- R¹: für Phenyl steht, das durch Halogen substituiert ist;
- R³: für Halogen steht.

Vorzugsweise ist der Phenylrest 1 bis 3-fach durch Halogen substituiert.

Hierbei ist unter Halogen Fluor, Chlor, Brom oder Iod, besonders bevorzugt Chlor zu verstehen.

Ganz besonders bevorzugt ist die Verbindung 2-Chloro-N-(4'-chloro-biphenyl-2-yl)-nicotinamid.

Unter den oben genannten Hydraten sind die entsprechenden Monohydrate besonders bevorzugt.

Die Herstellung der Amidverbindungen der Formel I oder II ist beispielsweise aus der EP-A-545 099 oder EP-A-589 301 bekannt oder kann nach analogen Verfahren erfolgen.

Gegenstand der vorliegenden Erfindung sind weiterhin Verfahren zur Herstellung der oben genannten Hydrate.

In einer Ausführungsform (Verfahren 1) umfasst das Verfahren die folgenden Schritte:
a) Lösen des Anhydrates von I in einem wasserlöslichen organischen Lösungsmittel; und
b) Präzipitation des Hydrates von I durch Zugabe von Wasser.

Hierbei ist unter dem Begriff wasserlösliches Lösungsmittel ein Lösungsmittel zu verstehen, dass zu mindestens 5% in Wasser löslich ist.

Das Verhältniss Wasser/organische Phase beträgt in Schritt a) des oben genannten Verfahrens im allgemeinen 1:10 bis 10:1, bevorzugt 1:3 bis 3:1.

Hierbei können als wasserlösliche organische Lösungsmittel cyclische Ether wie Dioxan oder Tetrahydrofuran, Alkohole wie Methanol, Ethanol, Propanol, Butanol oder Pentanol, sowie Dimethylformamid oder N-Methylpyrrolidon oder Mischungen der vorgenannten Lösungsmittel verwendet werden. Bevorzugt ist die Verwendung von Tetrahydrofuran.

In einer weiteren Ausführungsform (Verfahren 2) umfasst das Verfahren die folgenden Schritte:
a) Vermischen einer das Anhydrat von I enthaltenden organischen Lösung mit Wasser;
b) Erhitzen der in Schritt a) hergestellten Mischung auf eine Temperatur von 30-150°C;
c) Abkühlen der hergestellten Lösung.

Das Verhältniss Wasser/organische Phase beträgt in Schritt a) des oben genannten Verfahrens im allgemeinen 10:1 bis 1:10, bevorzugt 1:3 bis 3:1.

Hierbei können als organische Lösungsmittel cyclische Ether wie Dioxan oder Tetrahydrofuran, Ketone wie Aceton, Cyclohexanon oder MEK oder aromatische Lösungsmittel wie Benzol, Toluol, Xylol oder Lösungsmittel wie Dimethylformamid oder N-Methylpyrrolidon oder Mischungen der vorgenannten Lösungsmittel verwendet werden.

In einer bevorzugten Ausführungsform von Verfahren 2 wird die Lösung in Schritt b) bevorzugt bei einer Temperatur von 30-70°C, besonders bevorzugt von 30-60°C inkubiert und im Anschluß entsprechend abgekühlt.

In einer weiteren Ausführungsform (Verfahren 3) umfasst das Verfahren die folgenden Schritte:
a) Vermischen des festen Anhydrates von I mit Wasser; und
b) Erhitzen der in Schritt a) hergestellten Mischung auf eine Temperatur von 30-150°C bis das Anydrat zu in das Hydrat umgewandelt ist; oder
c) Inkubation der in Schritt a) hergestellten Mischung, wobei diese Scherkräften ausgesetzt wird, bis das Anydrat zu in das Hydrat umgewandelt ist.

In einer bevorzugten Ausführungsform des oben genannten Verfahrens wird die Lösung in Schritt b) bevorzugt bei einer Temperatur von 30-70°C, besonders bevorzugt von 30-60°C inkubiert und im Anschluß entsprechend abgekühlt.

Hierbei liegt die Inkubation in Schritt b) in einem Zeitraum von 30 min bis 48 h. Die Umwandlung von Anydrat in das Hydrat erfolgt zu mindestens 50% bevorzugt mindestens 70%.

Die Inkubation in Schritt c) wird mindestens über einen Zeitraum von 30 min bis 48 Stunden durchgeführt.

Das in Schritt c) beschriebene Aussetzen der Mischung mit starken Scherkräften kann durch Mahlung mittels geeigneter Mühlen erfolgen. Besonders bewährt haben sich hierbei Mühlen mit kurzer Verweilzeit des Produktes wie Rotor-Stator-Mühlen.

In beiden der oben genannten Verfahren kann der Umwandlungsgrad vom Anhydrat in das Hydrat mittels geeigneter Analytik wie z.Bsp IR-Spektroskopie oder Röntgen-Pulverdiffraktometrie überprüft werden.

Die Umwandlung von Hydrat/Anhydrat kann mittels IR kann beispielsweise durch die Verschiebung charakteristischer Banden (z.Bsp. C=O Streckschwingung) quantifiziert werden. So verschiebt sich beispielsweise die C=O Streckschwingung vom 2-Chloro-N-(4'-chloro-biphenyl-2-yl)-nicotinamidanhydrat zum 2-Chloro-N-(4'-chloro-biphenyl-2-yl)-nicotinamidmonohydrat von von 1650cm⁻¹ auf 1660cm⁻¹. Das Verschwinden der Bände bei 1650cm⁻¹ weist somit auf eine vollständige Umwandlung von Anhdrat zum Hydrat hin.

In Verfahren 3 kann die Herstellung des jeweiligen Hydrates nach einem der oben genannten Verfahren bereits in Gegenwart von Hilfstoffen erfolgen (sog. Direktverfahren).

Gegenstand der vorliegenden Erfindungen sind auch Suspoemulsionen (SE) bzw. Suspensionskonzentrate (SC) enthaltend als wesentliche Komponente das Hydrat eines Wirkstoffes I, wobei vorzugsweise A=A2 ist.

Zur Herstellung von SCs wird beispielsweise das Hydrat des Nicotinsäureanilid-Derivates in Gegenwart von Tensiden, gegebenenfalls weiterer Hilfstoffe in Wasser vermahlen.

In einer möglichen Ausführungsform der oben genannten Herstellungsweise werden zunächst die Tenside zusammen mit dem Frostschutzmittel und Wasser homogenisiert und dann ein nach dem erfindungsgemäßen Verfahren hergestelltes Hydrat des Nicotinsäureanilid-Derivates hinzugefügt. Die erhaltene Maische kann anschlißend direkt in einer Mühle z.Bsp. Rührwerks-Kugelmühle zerkleinert werden. Weitere geeignete Mühlen sind in Mollet, H. und Grubemann, A. "Formulierungstechnik", WILEY-VCH, 2000 erwähnt. Um die gewünschte Feinheit zu erreichen, kann es erforderlich sein, die Mühlenpassage mehrmals zu wiederholen.

Ist die gewünschte Kornverteilung von vorzugsweise von 40% kleiner als 2 Mikron und 100% kleiner als 12 Mikron erreicht, kann die Suspension in der Regel mit thixotrophen Agentien versetzt werden.

In einer bevorzugten Ausführungsform wird das Hydrat über die Schritte a) und b) des Verfahrens 3 hergestellt, wobei die entstehende Mischung zuvor mit entsprechenden Hilfstoffen versetzt worden ist. Im direkten Anschluß wird die hergestellte Mischung über wie oben beschrieben fein vermahlen. Hierbei ist das Vermahlen über eine Rührwerkskugelmühle besonders bevorzugt.

Für die oben genannten Formulierungen geeignete Tenside sind ionische Tenside und nichtionische Tenside, bevorzugt sind Gemische aus beiden.

Geeignete ionische Tenside sind beispielsweise Alkylarylsulfonate, Phenylsulfonate, Alkylsulfate, Alkylsulfonate, Alkylethersulfate, Alkylarylethersulfate, Alkylpolyglykoletherphosphate, Polyarylphenyletherphosphate, Alkylsulfosuccinate, Olefinsulfonate, Paraffinsulfonate, Petroleumsulfonate, Tauride, Sarkoside, Fettsäuren, Alkylnaphthalinsulfonsäuren, Naphthalinsulfonsäuren, Ligninsulfonsäuren, Kondensationsprodukte sulfonierter Naphthaline mit Formaldehyd oder mit Formaldehyd und Phenol und gegebenenfalls Harnstoff, Lignin-Sulfit-Ablauge, einschließlich ihrer Alkali-, Erdalkali-, Ammonium- und Amin-Salze, Alkylphosphate, quartäre Ammoniumverbindungen, Alkylphosphate, Aminoxide, Betaine und deren Gemische sowie Polycarboxylate wie z.Bsp. Polyacrylate, Maleinsäureanhydrid/Olefin-Copolymere (z.Bsp. Sokalan®CP9, BASF).

Bevorzugt sind Kondensationsprodukte sulfonierter Naphthaline oder Phenole mit Formaldehyd und gegebenenfalls Harnstoff, die als wasserlösliche Salze wie z.Bsp. als Natriumsalz vorliegen, wie Naphthalinsulfonsäure-Formaldehyd Kondensationsprodukte oder Kondensationsprodukte aus Phenolsulphonsäure, Formaldehyd und Harnstoff vorliegen (z.Bsp. Verbindungen wie Wettol®D1, Tamol®NN, Tamol®NH der Firma BASF oder Morwet®D425 der Firma Witco) .

Geeignete nichtionische Tenside sind beispielsweise Alkylphenolalkoxylate, Alkoholalkoxylate, Fettaminalkoxylate, Polyoxyeethylenglycerolfettsäureester, Rizinusölalkoxylate, Fettsäurealkoxylate, Fettsäureamidalkoxylate, Fettsäurepolydiethanolamide, Lanolinethoxylate, Fettsäurepolyglykolester, Isotridecylalkohol, Fettsäureamide, Methylcellulose, Fettsäureester, Silicon-Öle, Alkylpolyglykoside, Glycerolfettsäureester, Polyethylenglykol, Polypropylenglykol, Polyethylenglykolpolypropylenglykol-Blockcopolymere, Polyethylenglykolalkylether, Polypropylenglykolalkylether, Polyethylenglykolpolypropylenglykolether-Blockcopolymere und deren Gemische.

Bevorzugt sind Polyethylenglykolpolypropylenglykol-Blockcopolymere, Polyethylenglykolalkylether, Polypropylenglykolalkylether, Polyethylenglykolpolypropylenglykolether-Blockcopolymere (z. Bsp. Verbindungen der Pluronic®-Serie der BASF), Fettsäureakoholalkoxylate (z.Bsp. Verbindungen der Plurafac®-Serie von BASF, Atlas®G5000 der Uniqema oder Witoconol® NS500 der Crompton/Witco) und deren Gemische.

Bevorzugte Gemische von ionischen und nichtionischen Tensiden bestehen aus Polyethylenglykolpolypropylenglykolether-Blockcopolymere und/oder Fettsäurealkoxylate zusammen mit Kondensationsprodukten aus sulfonierten Phenole mit Harnstoff und Formaldehyd bzw. Naphthalinsulfonsäure-formaldehyd-Kondensationsprodukte (z.Bsp. Verbindungen wie Wettol®D1, Tamol®NN, Tamol®NH der Firma BASF oder Morwet®D425 der Firma Witco).

Für die oben genannten Formulierungstypen geeignete die Viskosität verändernde Additive sind Verbindungen, die der Formulierung ein pseudoplastisches Fließverhalten verleihen, d.h. hohe Viskosität im Ruhezustand und niedrige Viskosität im bewegten Zustand.

Geeignete Verbindungen sind beispielsweise Polysaccharide bzw. organische Schichtmineralien wie Xanthan Gum (Kelzan® der Fa. Kelco), Rhodopol® 23 (Rhone Poulenc) oder Veegum® (Firma R.T. Vanderbilt) oder Attaclay® (Firma Engelhardt) .

Als für die erfindungsgemäßen Formulierungen geeignete Antischaummittel kommen beispielsweise Silikonemulsionen (wie z.Bsp. Silikon® SRE, Firma Wacker oder Rhodorsil® der Firma Rhodia), langkettige Alkohole, Fettsäuren, fluororganische Verbindungen und deren Gemische in Betracht.

Bakterizide können zur Stabilisierung der wäßrigen Fungizid-Formulierung zugesetzt werden. Geeignete Bakterizide sind beispielsweise Proxel® der Fa. ICI oder Acticide® RS der Fa. Thor Chemie und Kanthon® MK der Firma Rhom & Haas.

Geeignete Frostschutzmittel sind z.Bsp. Ethylenglycol, Propylenglycol oder Glycerin.

Zur Vergrößerung des Wirkungsspektrums oder zur Erzielung besonderer Effekte, z.B. zusätzlicher Schutz gegen Insekten, Spinnentiere oder Milben können die vorstehend genannten Formulierungen mit weiteren agrochemischen Wirkstoffen kombiniert werden, die im letzteren Fall mit geeigneten Additiven eingearbeitet werden können. Hierbei ist unter dem Begriff Additiv eine Auswahl aus den oben genannten Tensiden und weiteren Hilfstoffen zu verstehen. Bei einer SC-Formulierung können zusätzliche Wirkstoff in der wässrigen Phase gelöst oder in fein vermahlener Form suspendiert vorliegen. Im Falle einer SE-Formulierung ist in dem erfindungsgemäßen SC ein weiterer Wirkstoff in flüssiger oder gelöster Form emulgiert in der Formulierung neben dem suspendierten Wirkstoff vorhanden.

Für die organische Phase der erfindungsgemäßen SE-Formulierungen sind aromatische Kohlenwasserstoffe auf Alkylbenzolbasis wie z.B. Xylol, Toluol, Trimethylbenzol, Methylethylbenzol, Dimethylethylbenzol, Diethylbenzol, Tetramethylbenzol und Pentamethylbenzol geeignet. Besonders geeignet sind Gemische von aromatischen Kohlenwasserstoffen, wie die mit der Bezeichnung Solvesso® (Hersteller Esso) oder Shellsol® (Hersteller Shell) im Handel erhältlichen Lösungsmittel. Als Lösungsmittel auf rein aliphatischer Basis können Päraffinöl (z.B. Linpar®: C₁₄-C₁₇-Kohlenwasserstoff-Fraktion der Fa. Wintershall), aber auch native Öle wie Rapsöl und Sojaöl zum Einsatz kommen, soweit sie den Wirkstoff ausreichend zu lösen vermögen. Desweiteren können Ester natürlicher und synthetischer Fettsäuren oder Polycarbonsäuren sowie deren Gemische (z.Bsp. Acetate wie Methylacetat, Ethylacetat, Propylacetat aber auch Acetate von höherkettigen Alkoholen (C₅-C₂₀-Alkohle), Dialkyladipat, Alkyl-glutarat oder Alkylcitrat) verwendet werden.

Unter dem Begriff des agrochemischen Wirkstoffes sind im Rahmen der vorliegenden Erfindung sowohl Fungizide als auch Insektizide und Wachstumsregulatoren zu verstehen.

Die folgende Liste von Fungiziden zeigt mögliche Wirkstoffe auf:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4, 6-dinitrophenyl-3,3-dimethylacrylat, 2-sec-Butyl-4, 6-dinitrophenyl-isopropylcarbonat, 5-Nitro-isophthalsäuredi-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1- [bis- (dimethylamino) -phosphinyl]-3-phenyl-1,2,4- triazol, 2,3-Dicyano-1,4-dithioanthrachinon, 2-Thio-1, 3-dithiolo [4, 5-b] chinoxalin, 1-(Butylcarbamoyl)-2-benzimidazol-carbaminsäuremethylester, 2-Methoxycarbonylamino-benzimidazol , 2-(Furyl-(2))-benzimidazol , 2-(Thiazolyl-(4))-benzimidazol, N-(1,1,2,2-Tetrachlorethylthio) -tetrahydrophthalimid, N-Trichlormethylthio-tetrahydrophthalimid, N-Trichlormethylthiophthalimid, N-Dichlorfluormethylthio-N',N'-dimethyl-N-phenylschwefelsäure-diamid, 5-Ethoxy-3-trichlormethyl-1,2,3-thiadiazol, 2-Rhodanmethylthiobenzthiazol, 1,4-Dichlor-2,5-dimethoxybenzol, 4- (2-Chlorphenylhydrazono) -3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5, 6-dihydro-4H-pyran-3-carbonsäure-anilid, 2-Methyl-furan-3-carbonsäureanilid, 2,5-Dimethyl-furan-3-carbonsäureanilid, 2 , 4, 5-Trimethyl-furan-3-carbonsäureanilid, 2,5-Dimethyl-furan-3-carbonsäurecyclohexylamid, N-Cyclohexyl-N-methoxy-2, 5-dimethyl-furan-3-carbonsäureamid, 2-Methyl-benzoesäure-anilid, 2-Iodbenzoesäure-anilid, N-Formyl-N-morpholin-2,2,2-trichlorethylacetal, Piperazin-1,4-diylbis-1-(2 , 2 , 2-trichlorethyl) -formamid, 1-(3,4-Dichloranilino)-1-formylamino-2, 2 , 2-trichlorethan, 2, 6-Dimethyl-N-tridecyl-morpholin bzw. dessen Salze, 2,6-Dimethyl-N-cyclododecyl-morpholin bzw. dessen Salze, N- [3- (p-tert.-Butylphenyl)-2-methylpropyl]-cis-2,6-dimethyl-morpholin, N-[3-(p-tert.-Butylphenyl)-2-methylpropyl]-piperidin, 1- [2- (2, 4-Dichlorphenyl)-4-ethyl-1,3-dioxolan-2-yl-ethyl]-1H-1,2,4-triazol, 1-[2-(2,4-Dichlorphenyl) -4-n-propyl-1, 3-dioxolan-2-yl-ethyl ] -1H-1, 2 , 4-triazol, N- (n-Propyl) -N- (2,4, 6-trichlorphenoxyethyl) -N'-imidazol-ylharnstoff, 1- (4-Chlorphenoxy) -3, 3-dimethyl-1- (1H-1, 2, 4-triazol-1-yl) -2-butanon, 1-(4-Chlorphenoxy) -3 , 3-dimethyl-1- (1H-1, 2, 4-triazol-1-yl)-2-butanol, (2RS, 3RS) -1- [ 3- (2-Chlorphenyl)-2-(4-fluorphenyl)-oxiran-2-ylmethyl]-1H-1,2,4-triazol, α-(2-Chlorphenyl)-α-(4-chlorphenyl) -5-pyrimidin-methanol , 5-Butyl-2-dimethylamino-4-hydroxy-6-methyl-pyrimidin, Bis-(p-chlorphenyl) -3-pyridinmethanol, 1,2-Bis-(3-ethoxycarbonyl-2-thioureido)-benzol, 1,2-Bis-(3-methoxycarbonyl-2-thioureido) -benzol, Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl)-anilin,
Phenylpyrrole wie 4-(2,2-Difluor-1,3-benzodioxol-4-yl)-pyrrol-3-carbonitril,
Zimtsäureamide wie 3-(4-Chlorphenyl)-3-(3,4-dimethoxyphenyl)-acrylsäuremorpholid,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl-ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin- 2,4-dion, 3-(3,5-Dichlorphenyl)-1-isopropylcarbamoylhydantoin, N-(3,5-DichlorphenyD-1,2-dimethylcyclopropan-1, 2-dicarbonsäureimid, 2-Cyano-[N-(ethylaminocarbonyl) -2-methoximino] -acetamid, 1-[2-(2,4-Dichlorphenyl)-pentyl]-1H-1,2, 4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol, 5-Chlor-2-cyano-4-p-tolyl-imidazol-1-sulfonsäuredimethylamid, 3, 5-Dichlor-N- (3-chlor-1-ethyl-1-methyl-2-oxo-propyl) -4-methylbenzamid.
Strobilurine wie Methyl-E-methoxyimino-[a-(o-tolyloxy)-o-tolyl] acetat, Methyl-E-2-{2-[6-(2-cyanophenoxy)-pyrimidin-4-yloxy]-phenyl}-3-methoxyacrylat (azoxystrobin), Methyl-E-methoxyimino-[a-(2-phenoxyphenyl)]-acetamid (Metominostrobin) , Methyl-E-methoxyimino-[a-(2,5-dimethylphenoxy)-o-tolyl]-acetamid, Methyl-E-2-{2-[2 -trifluormethylpyridyl-6-}oxymethyl]-phenyl}3-methoxyacrylat, (E, E) -Methoximino-{2-[1- (3-trifluormethylphenyl)-ethylidenaminooxymethyl]-phenyl)-essigsäuremethylester (trifloxystrobin), Methyl-N-(2-{[1-(4-chlorphenyl)-1H-pyrazol-3-yl] oxymethyl}phenyl) N-methoxy-carbamat.

Die folgende Liste von Insektiziden zeigt mögliche Wirkstoffe auf:
Neonicotinoide/Chlornicotinyl-Verbindungen (wie Imidacloprid, Acetamiprid, Nitenpyram, Thiacloprid; Thiamethoxam, MIT-446 (Terafuranitdine)
Pyrrole (wie Chlorphenapyr, Fludioxonil)
Organophosphate (wie Acephate, Azinphos-methyl, Chlorpyrifos, Dimethoate, Disulfoton Fosthiazate, Methamidophos, Methidathion, Methyl-Parathion, Oxydemeton-methyl, Phorate, Phosalone, Phosmet, Profenofos, Trichlorfon, Malathion, Phosphamidon, Monocrotophos, Fenitrothion, Diazinon, EPN)
Carbamate (wie Alanycarb, Aldicarb, Benfuracarb, Carbofuran, Carbosulfan, Furathiocarb, Methomyl, Oxamyl, Pirimicarb, Thiodicarb, Fenobucarb)
Pyrethroide (wie Bifenthrin, Cyfluthrin, Cypermethrin, Deltamethrin, Ethofenprox, Esfenvalerate, Fenpropathrin, Flucythrinate, Fluvalinate, Lambda-Cyhalothrin, Permethrin, Pyrethrin I, Pyrethrin II, Silafluofen, Tau-Fluvalinate, Tralomethrin, Zeta-Cypermethrin)
Harnstoffderivate (wie Diflubenzuron, Flucycloxuron, Flufenoxuron, Hexaflumuron, Lufenuron, Novaluron, Triflumuron
Juvenuide (wie Buprofezin, Diofenolan,Fenoxycarb, Pyriproxifen, Methoxyfenozide, Tebufenozide)

Die folgende Liste von Verbindungen mit wachstumsregulatorischer Wirkung zeigt mögliche Wirkstoffe auf:
1-Naphthylacetamid, 1-Naphthylessigsäure, 2-Naphthyloxyessigsäure, 3-CPA, 4-CPA, Ancymidol, Anthrachinon, BAP, Butifos; Tribufos, Butralin, Chlorflurenol, Chlormequat, Clofencet, Cyclanilide, Daminozide, Dicamba, Dikegulac sodium, Dimethipin, Chlorfenethol, Etacelasil, Ethephon, Ethychlozate, Fenoprop, 2,4,5-TP, Fluoridamid, Flurprimidol, Flutriafol, Gibberellic acid, Gibberillin, Guazatin, Imazalil, Indolylbuttersäure, Indolylessigsäure, Karetazan, Kinetin, Lactidichlor-ethyl, Maleic hydrazide, Mefluidide, Mepiquat-chlorid, Naptalam, Paclobutrazole, Prohexadione calcium, Quinmerac, Sintofen, Tetcyclacis, Thidiazuron, Triiodobezoicacid, Triapenthenol, Triazethan, Tribufos, Trinexapacethyl,Uniconazole.

Weiterer Gegenstand der vorliegenden Erfindung ist ein Verfahren zur Bekämpfung von phytopathogenen Pilzen, unerwünschtem Insekten- oder Milbenbefall und/oder zur Regulation des Wachstums von Pflanzen, welches darauf basiert, daß man eine eine erfindungsgemäße SC bzw. SE Formulierung entsprechend verdünnt und auf den jeweiligen Schädling oder Pflanze appliziert, wobei die SC- bzw. SE-Formulierung jeweils einen weiteren der oben erwähnten agrochemischen Wirkstoffe enthalten kann. Die Verdünnung richtet sich hierbei nach Art des Wirkstoffes bzw. Wirkstoffkombination.

Unter phytopathogenen Pilzen bekämpfbar durch die erfindungsgemäßen Formulierungen sind beispielsweise folgende Spezies zu verstehen:
*Alternaria*-Arten, *Podosphaera*-Arten, *Sclerotinia*-Arten, *Physalos*pora *canker* an Gemüse und Obst, *Botrytis cinerea* (Grauschimmel) an Erdbeeren, Gemüse, Zierpflanzen und Reben, *Corynespora melonis* an Gurken, Erdbeeren; *Colletotrichum-*Arten an Gurken; *Diplocarpon rosae* an Rosen; *Elsinoe fawcetti* und *Diaporthe citri* an Citrus-Früchten; *Sphaerotheca*-Arten an Gurken, Kürbisgewächsen, Erdbeeren und Rosen; *Cercospora*-Arten an Erdnüssen, Zuckerrüben, Eierpflanzen und Dattelpflaumen; *Erysiphe cichoracearum* und *Sphaerotheca fuliginea* an Kürbisgewächsen, *Leveiillina taurica* an Piment; *Mycoshpaerella-Arten* an Äpfeln und Japanischer Aprikose; *Phyllactinia kakicola, Gloesporium kaki*, an Japanischer Aprikose; *Gymnosporangium yamadae, Leptotthrydium pomi, Podosphaera leucotricha* und *Gloedes pomigena* an Äpfeln; *Cladosporium carpophil um* an Birnen und Japanischer Aprikose; *Phomopsis*-Arten an Birnen; *Phytopora-Arten* an Citrus Früchten, Kartoffeln, Zwiebeln; *Phytophthora infestans* an Kartoffeln und Tomaten, *Erysiphe graminis* (echter Mehltau) an Getreide, *Fusarium-* und *Verticillium-*Arten an verschiedenen Pflanzen, *Glomerella cingulata* an Tee; *Helminthosporiumi-*Arten an Getreide, *Mycosphaerella*-Arten an Bananen und Erdnüssen, *Plasmopara viticola* an Reben und Grapefruits, *Personospora*-Arten an Zwiebeln, Spinat und Chrysantemen; *Phaeoisariopsis* vitis und *Spaceloma ampelina* an Grapefruits; *Pseudocercosporella herpotrichoides* an Weizen und Gerste, *Pseudoperonospora*-Arten an Hopfen und Gurken, *Puccinia*-Arten und *Typhula*-Arten an Getreide, *Pyricularia* oryzae an Reis, *Rhizoctonia*-Arten an Baumwolle, Reis und Rasen, *Septoria nodorum* an Weizen, *Uncinula necator* an Reben, *Ustilago*-Arten an Getreide und Zuckerrohr, sowie *Venturia*-Arten (Schorf) an Äpfeln und Birnen.
Unter Insekten bekämpfbar durch die erfindungsgemäßen Formulierungen sind beispielsweise Insekten aus der Ordnung Lepidoptera (Schmetterlinge und Motten) beispielsweise *Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithotis blancardella, Lobesia botrana, Loxostege sticticalis, Lymantria dispar, Lymantria monacha, Lyonetia clerkella, Malacosoma neustria*, *Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Si totroga cerealella, Sparganothis pilleriana, Spodoptera frugiperda, Spodoptera littoralis*, *Spodoptera litura*, *Thaumatopoea pityocampa, Tortrix viridana, Trichoplusia ni* und *Zeiraphera canadensis,*
aus der *Ordnung Coleoptera* (Käfer), z.B. *Agrilus sinuatus, Agriotes lineatus, Agriotes obscurus, Amphimallus solstitialis,* An*isandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp. , Phyllopertha horticola, Phyllotreta nemorum, Phyllotreta striolata, Popillia japonica, Sitona lineatus und Sitophilus granaria,*
aus der Ordnung der Diptera (Zweiflügler), z.B. *Aedes aegypti, Aedes vexans, Anastrepha ludens, Anopheles maculipennis, Ceratitis capitata, Chrysomya bezziana, Chrysomya hominivorax, Chryso* *mya macellaria, Contarinia sorghicola, Cordylobia anthropophaga*, *Culex pipiens, Dacus cucurbitae, Dacus oleae, Dasineura brassicae*, *Fannia canicularis*, *Gasterophilus* intestinalis, *Glossina morsitans, Haematobia irritans, Haplodiplosis equestris*, *Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia anti qua, Phorbia brassi cae, Phorbia coarctata, Rhagoletis cerasi, Rhagoletis pomonella, Tabanus bovinus, Tipula oleracea und Tipula paludosa,*
aus der *Ordnung Thysanoptera,* z.B. *Frankliniella fusca, Frankliniella occiden talis, Franacliniella tritici, Scirtothrips citri, Thrips oryzae, Thrips palmi* und *Thrips tabaci,*
aus der *Ordnung Hymenoptera,* z.B. *Athalia rosae, Atta cephalotes, Atta sexdens, Atta texana, Hoplocampa minuta, Hoplocampa testudinea, Monomorium pharaonis, Solenopsis geminata und Solenopsis invicta,*
aus der *Ordnung Heteroptera,* z.B. *Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor,*
aus der Ordnung *Homoptera,* z.B. *Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum* und *Viteus vitifolii,*
aus der Ordnung *Isoptera,* z.B. *Calotermes flavicollis, Leucotermes flavipes, Reticulitermes lucifugus und Termes natalensis,*
aus der Ordnung *Orthoptera,* z.B. *Acheta domestica, Blatta orientalis, Blattella germanica, Forficula auricularia, Gryllotalpa gryllotalpa, Locusta migratoria, Melanoplus bivittatus, Melano* *plus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus* und *Tachycines asynamorus,*
aus der *Ordnung Acari,* z.B. *Amblyomma americanum, Amblyomma variegaturn, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata*, *Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius* und *Tetranychus urticae,*
aus der Ordnung der Nematoden wie Wurzelgallennematoden, z.B. *Meloidogyne hapla, Meloidogyne incognita, Meloidogyne javanica,* Zysten bildende Nematoden, *z.B. Globodera rostochiensis, Heterodera avenae, Heterodera glycines, Heterodera schachtii, Heterodera trifolii, Stöck- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus* und *Pratylenchus goodeyi.*

Die Regulation des Wachstums von Pflanzen kann durch die bereits weiter oben erwähnten Wachstumsregulatoren erfolgen oder durch den Einsatz von Dünger.

Eine bevorzugte Ausführungsform des oben genannten Verfahrens ist ein Verfahren zur Bekämpfung von phytopathogenen Pilzen.

Alle oben genannten Verfahren können dadurch realisiert werden, daß man eine eine erfindungsgemäße SC bzw. SE Formulierung entsprechend verdünnt und auf den jeweiligen Schädling oder die vor dem jeweiligen Schädling zu schützenden Materialen, Plfanzen, Boden und Saatgüter appliziert an sich appliziert, wobei die jeweilige SC oder SE-Formulierung noch einen weiteren fungiziden Wirkstoff enthalten kann.

Hierbei kann die Applikation der erfindungsgemäßen Zusammensetzugen bzw. der Substanzen kann im Vorauflauf- oder im Nachauflaufverfahren erfolgen. Sind die entsprechenden Wirkstoffe für gewisse Kulturpflanzen weniger verträglich, so können Ausbringungstechniken angewandt werden, bei welchen die verdünnten SC- bzw. SE-Formulierungen mit Hilfe der Spritzgeräte so gespritzt werden, daß die Blätter der empfindlichen Kulturpflanzen nach Möglichkeit nicht getroffen werden, während die agrochemischen Wirkstoffe auf die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha.

Im folgenden wird das erfindungsgemäße Verfahren anhand von Beispielen erläutert:

### Beispiel 1

### A) Herstellen des Anhydrates

2-Chloro-N-(4'-chloro-biphenyl-2-yl)-nicotinamid wurde am Ende der Synthese als heiße xylolische Lösung erhalten. Bei langsamem Abkühlen kristallisierte der Wirkstoff aus Xylol nahezu vollständig aus. Nach Filtration wurde durch Trocknen im Vakuumtrockenschrank restliches Xylol entfernt, wodurch der Wirkstoff in Form des Anhydrates anfiel. Die physikalischen Eigenschaften sind in Tabelle 1 zusammengefasst.

### B) Herstellen des Hydrates

1g 2-Chloro-N-(4'-chloro-biphenyl-2-yl)-nicotinamidanhydrates wurde bei 40°C in 20 ml THF gelöst und im Anschluß in 20 ml Wasser gegossen. Das ausgefallene Material wurde abgesaugt und bei 40°C im Trockenschrank getrocknet. Die physikalischen Eigenschaften des so hergestellten 2-Chloro-N-(4'-chloro-biphenyl-2-yl)-nicotinamid Monohydrates sind in Tabelle 1 wiedergegeben.

### C) Analytik der Umwandlung Hydrat/Anhydrat

Die IR Spektren wurden mit einem FT-IR Spektrometer aufgenommen. Die Proben wurden entweder als KBr-Presslinge präpariert oder mit einer sogenannten Single-Reflexions-Diamant-ATR-Messeinheit (Durascope, Firma Resultec) vermessen.

Bei der Vermessung mittels der Single-Reflexions-Diamant-ATR-Messeinheit wurde die den zu untersuchenden Feststoff enthaltende Suspension auf eine Tonscherbe oder ein Filterpapier appliziert. Der nach der Entfernung des Wassers verbleibende Feststoff wurde im Anschluß auf die Single-Reflexions-Diamant-ATR-Messeinheit aufgetragen.

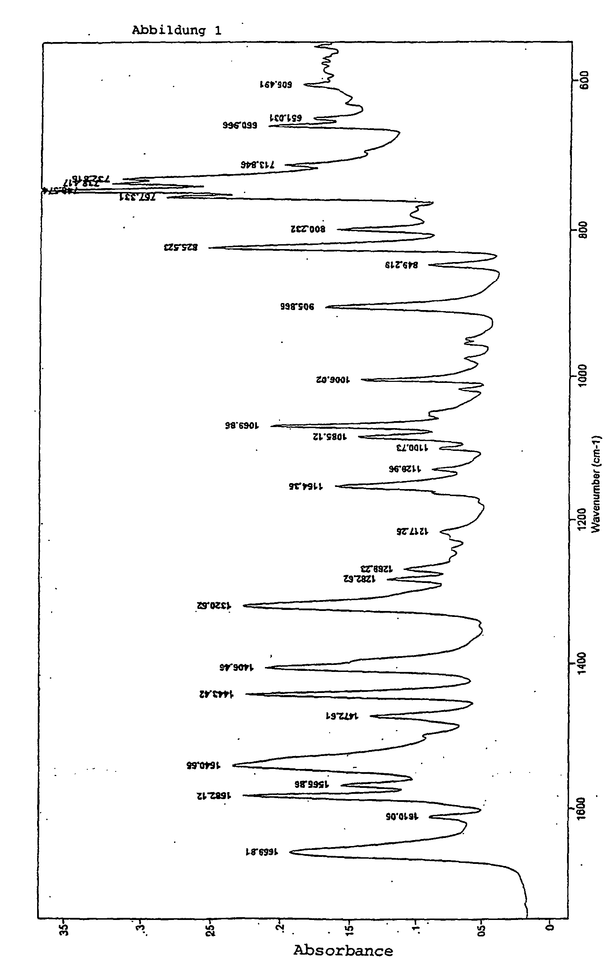

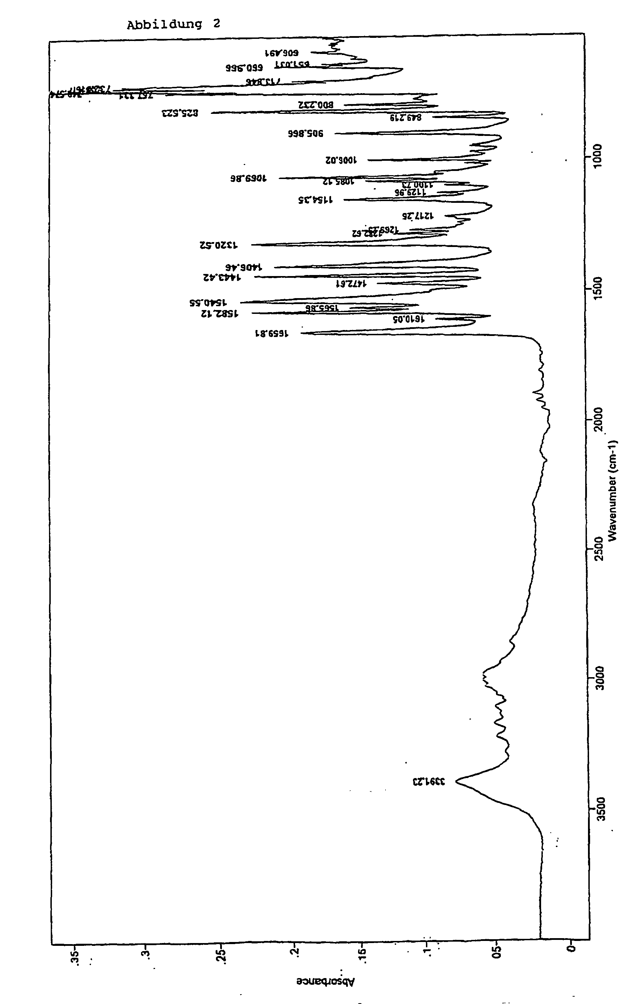

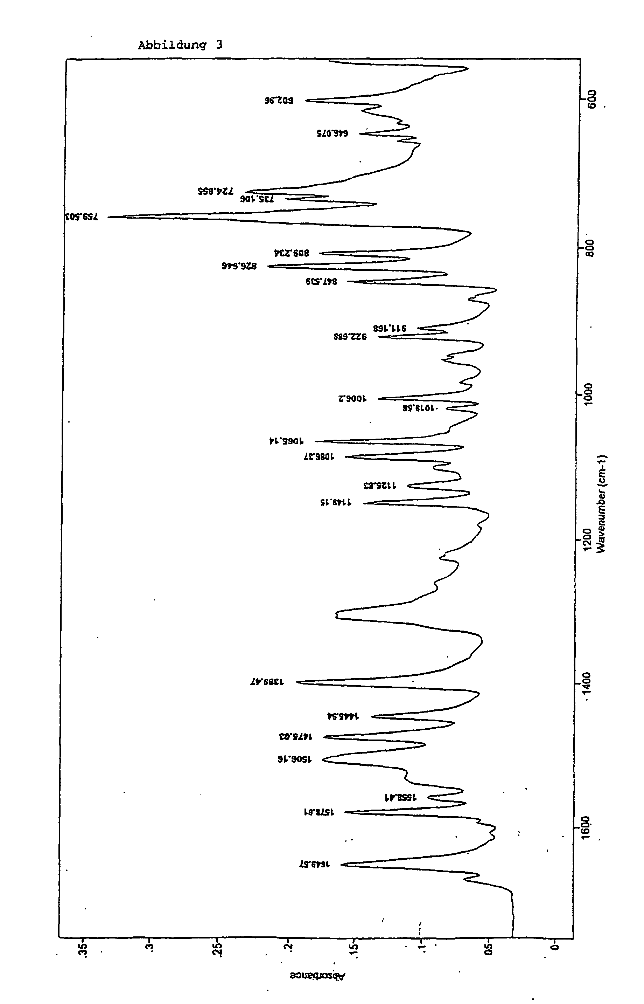

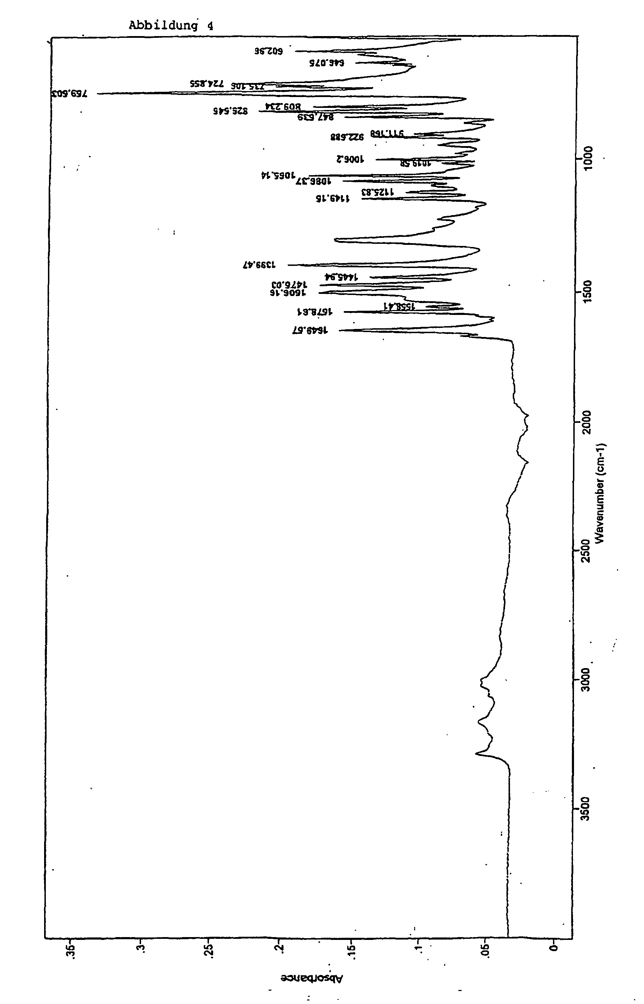

Die in den Abbildungen 1 bis 4 gezeigten IR-Spektren (s.a. Tabelle 1) verdeutlichen weiterhin die Unterschiede 2-Chloro-N-(4'-chloro-biphenyl-2-yl) -nicotinamidmonohydrates und 2-Chloro-N-(4'-chloro-biphenyl-2-yl)-nicotinamidanhydrates. IR-Spektroskopie kann somit für die Überprüfung der Umwandlungsgrades herangezogen werden.

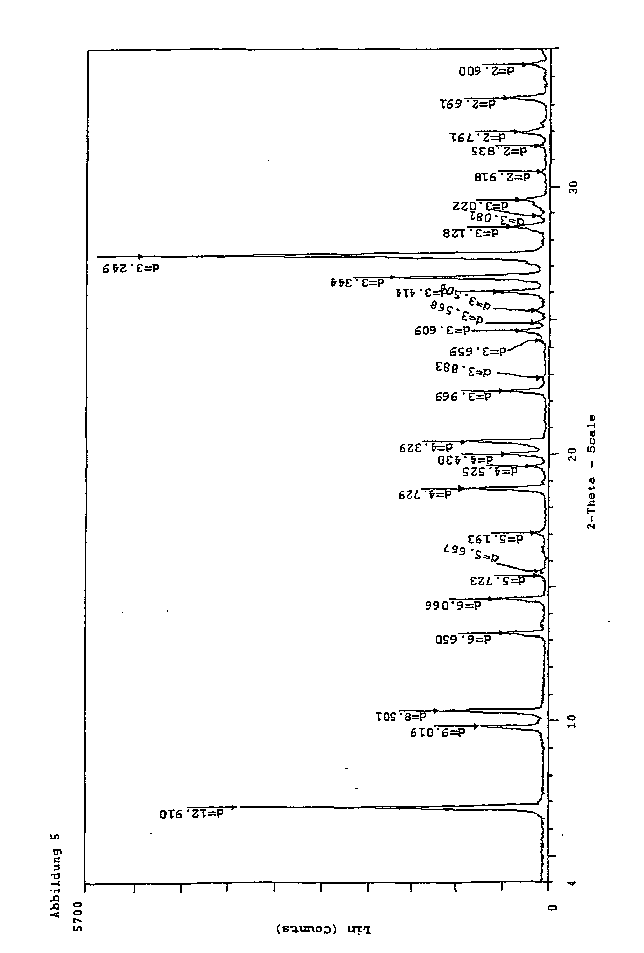

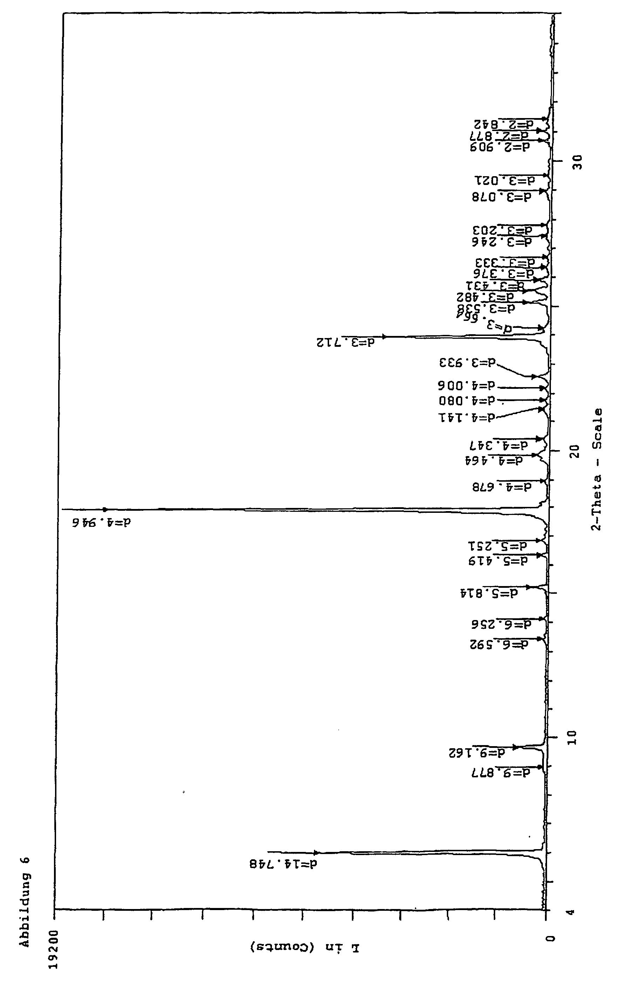

Die in den Abbildungen 5 bis 6 gezeigten Röntgenpulverdiagramme (s.a. Tabelle 1) wurden nach Standardmethoden aufgenommen, wie sie z.B. in H.P. Klug und L.E. Alexander, X-Ray Diffraction Procedures (1974) oder R. Jenkins und R.L. Snyder, X-Ray Powder Diffractometry (1996) beschrieben sind. Die Messungen wurden mit Cu-Kα-Strahlung an einem Siemens D-5000 Diffraktometer im Breich der Diffraktionswinkel 2θ = 4° - 35° mit einer Schrittweite von 0.02° durchgeführt.

**Tabelle 1**

| | **Anhydrat [C**_{**18**}**H**_{**12**}**Cl**_{**2**}**N**_{**2**}**O]** | **Hydrat [C**_{**18**}**H**_{**14**}**Cl**_{**2**}**N**_{**2**}**O**_{**2**}**]** |
|---|---|---|
| Molgewicht | 343,2 g/mol | 361,2 g/mol |
| Schmelzpunkt (DSC) | 145,2°C | endotherm 80-92°C weiterer Peak bei 145,2°C |
| Dichte | 1,42 g/mol | 1,43 g/mol |
| Röntgenreflexe (2θ Grad) Cu-Kα | 18; 22,5; 9,5; 6 | 27,2; 18,5; 10,5; 7 |
| IR-Absorption | 1650cm⁻¹ | 1660cm⁻¹, 3391cm⁻¹, 800cm⁻¹, |
| Wassergehalt | < 1% | 5% |

### Beispiel 2

Die Handelsnamen der für die Formulierung des Hydrates/Anhydrates aus Beispiel 1 verwendeten Hilfstoffe sind in Tabelle 2 zusammengefasst.

**Tabelle 2**

| **Name** | **chem. Bezeichnung** | **Bezugsquelle** |
|---|---|---|
| Silikon® SRE | Silikonölemulsion | Wacker Chemie |
| Wettol® D1 | Kondensationsprodukt ans Phenolsulfonsäure, Harnstoff und Formaldehyd | BASF Aktiengesellschaft |
| Pluxonic® PE 10500 | EO/PO-Blockcopolymer | BASF Aktiengesellschaft |
| Proxel® GXL | wässrige Dipropylenglycol Lösung enthaltend 20% 1,2-Benzisothiazolin-3-on | ICI |

### A) Formulierungen des Anhydrates

500 g des 2-Chloro-N-(4'-chloro-biphenyl-2-yl)-nicotinamid-anhydrates wurden zu einer Mischung von 30 g Wettol D1, 40 g Pluronic PE 10500, 4 g Proxel GXL, 50 g Propylenglykol und 5 g Silikon SRE in ca. 300 ml Wasser gegeben. Nach kurzem Rühren wurde die Mischung mit 2 l/h durch eine Dyno-Mühle KDL (1.2 mm Perlen, Mahlraum 1,21) gepumpt. Bereits nach kurzer Zeit verstopfte die Mühle und musste abgeschaltet werden. Die in der Vorlage aufgefangene Suspension begann sich ebenfalls zu verfestigen.

### B) Formulierungen des Hydrates

500 g des 2-Chloro-N-(4'-chloro-biphenyl-2-yl)-nicotinamidmonohy; drates wurden wie unter A) beschrieben zusammen mit Hilfsmitteln angesetzt. Nach kurzem Rühren wurde die Mischung mit 2 l/h durch eine Dyno-Mühle KDL (1.2 mm Perlen, Mahlraum 1,21) gepumpt. Der Mahlvorgang konnte ohne Probleme durchgeführt werden. Ein Verstopfen der Mühle oder eine Kristallisation im Vorlagebehälter konnte nicht beobachtet werden. Nach acht Mahlpassagen war eine ausreichende Teilchenfeinheit von 70 % < 2 µm (Malvern Mastersizer) erreicht, d.h. der Teilchendurchmesser von 70% der Teilchen ist < als 2 µm.

### C) Charakterisierung und Vergleich der hergestellten Formulierungen

Während bei der unter Beispiel 2 A) beschriebenen Vorgehensweise noch während der Herstellung ein lehmartiger Kristallbrei entstand (Formulierung A) , der verworfen werden musste, entstand in dem unter B) beschriebenen Verfahren (Formulierung B) eine lagerstabile Formulierung, in der keine weitergehende Kristallisation beobachtet wurde.

Die Messung der Partikelgröße der hergestellten Formulierung B wurde mit einem Malvern Mastersizer vorgenommen (s. Tabelle 3). Angegeben ist der prozentuale Anteil der Partikel kleiner als 2µm.

**Tabelle 3**

| | nach 0 h | nach 1 Monat [20°C] | nach 6 Monaten [20°C] | nach 1 Monat [40°C] |
|---|---|---|---|---|
| Teilchendurchmesser [%<2µm] | 79 | 79 | 79 | 75 |

### Beispiel 3

### Herstellung des Hydrates durch Direktumwandlung

### A) Mechanisches Verfahren

25 kg des des 2-Chloro-N-(4'-chloro-biphenyl-2-yl)-nicotinamidanhydrats werden zu einer Mischung von 1,5 kg Wettol® D1, 2 kg Pluronic® PE 10500, 200 g Proxel® GXL, 2,5 kg Propylenglykol und 250 g Silikon® SRE in ca. 15 1 Wasser gegeben.

Zur Umwandlung in das Hydrat wurde die Mischung mehrfach durch eine Rotor-Stator-Mühle vom Typ PuC (Spaltweite 0.2 mm, Durchsatz 200-300 l/h) gegeben. Nach jeder Passage wurde der Umwandlungsgrad vom Anhydrat in das Hydrat mittels IR-Spektroskopie überprüft. Nach 4-7 Passagen war die Umwandlung vollständig. Die anschliessend durchgeführte Feinmahlung mittels Dyno-Mühle verlief problemlos. Es wurde spezifikationsgerechte lagerstabile Ware erhalten.

### B) Thermisches Verfahren

25 kg des 2-Chloro-N-(4'-chloro-biphenyl-2-yl)-nicotinamid-anhydrates werden gemäss der unter 3 A) beschriebenen Rezeptur mit Hilfsmitteln und Wasser in einem beheizbaren Rührbehälter angesetzt. Die Mischung wurde bei 50°C mit einem Propellerrührer 5 h gerührt. Die Kontrolle mittels IR-Spektroskopie zeigte, dass nach diesem Zeitraum eine vollständige Umwandlung in das Hydrat erfolgt war. Nach Abkühlen auf 30°C erfolgte Mahlung in einer Rührwerks-Kugelmühle (Typ Dyno) und führte nach 8 Passagen bei einem Durchsatz von 100 l/h zu spezifikationsgerechtem Produkt.

Beschreibung der Abbildungen:
Abbildung 1: IR-Sprektrum von 2-Chloro-N-(4'-chloro-biphenyl-2-yl)-nicotinamidanhydrat (1800cm⁻¹ bis 600cm⁻¹)
Abbildung 2: IR-Sprektrum von 2-Chloro-N-(4'-chloro-biphenyl-2-yl)-nicotinamidanhydrat (4000cm⁻¹ bis 500cm⁻¹)
Abbildung 3: IR-Sprektrum von 2-Chloro-N-(4'-chloro-biphenyl-2-yl)-nicotinamidmonohydrates (1800cm⁻¹ bis 600cm⁻¹)
Abbildung 4: IR-Sprektrum von 2-Chloro-N-(4'-chloro-biphenyl-2-yl)-nicotinamidmonohydrates (4000cm⁻¹ bis 500cm⁻¹)
Abbildung 5: Röntgenpulverdiffraktogramm des 2-Chloro-N-(4'-chloro-biphenyl-2-yl)-nicotinamidmonohydrates [Bedingungen: Step: 0.020° - Step time: 4.5 s - WL1: 1.54056 - Temp.: 25°C (Room)]
Abbildung 6: Röntgenpulverdiffraktogramm des 2-Chloro-N-(4'-chloro-biphenyl-2-yl)-nicotinamidanhydrates [Bedingungen: Step: 0.020° - Step time: 4.5 s - WL1: 1.54056 - Temp.: 25°C (Room)]

## Patentansprüche

1. Kristalline Hydrate substituierter Anilid-Derivate der Formel I 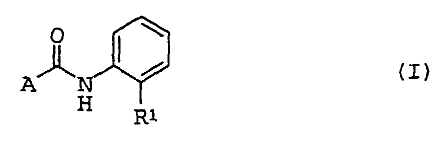 worin
A für 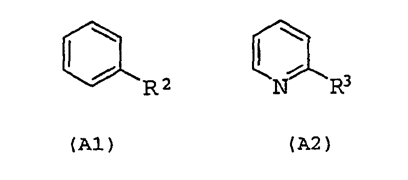
R¹ für Phenyl steht, das durch Halogen substituiert ist,
R² für Methyl, Difluormethyl, Trifluormethyl, Chlor, Brom oder Jod steht,
R³ für Trifluormethyl oder Chlor steht.

2. Hydrate nach Anspruch 1, die in Form der Monohydrate vorliegen.

3. Verfahren zur Herstellung von Hydraten nach Anspruch 1 oder 2, welches die folgenden Schritte umfasst:
a) Lösen des Anhydrates von I in einem wasserlöslichen organischen Lösungsmittel; und
b) Präzipitation des Hydrates von I durch Zugabe von Wasser.

4. Verfahren nach Anspruch 3, **dadurch gekennzeichnet, dass** man als organisches Lösungsmittel Dioxan, Tetrahydrofuran, Dimethylformamid oder N-Methylpyrrolidon oder Mischungen der vorgenannten Lösungsmittel verwendet.

5. Verfahren zur Herstellung von Hydraten nach Anspruch 1 oder 2, welches die folgenden Schritte umfasst:
a) Vermischen einer das Anhydrat von I enthaltenden organischen Lösung mit Wasser;
b) Erhitzen der in Schritt a) hergestellten Mischung auf eine Temperatur von 30-150°C;
c) Abkühlen der hergestellten Lösung.

6. Verfahren nach Anspruch 5, **dadurch gekennzeichnet, dass** man als organisches Lösungsmittel Benzol, Toluol, Xylol, Tetrahydrofuran, Dimethylformamid oder N-Methylpyrrolidon oder Mischungen der vorgenannten Lösungsmittel verwendet.

7. Verfahren zur Herstellung von Hydraten nach Anspruch 1 oder 2, welches die folgenden Schritte umfasst:
a) Vermischen des festen Anhyrates von I mit Wasser;
b) Erhitzen der in Schritt a) hergestellten Mischung auf eine Temperatur von 30-150°C bis das Anydrat in das Hydrat umgewandelt ist; oder
c) Inkubation der in Schritt a) hergestellten Mischung, wobei diese Scherkräften ausgesetzt wird, bis das Anydrat in das Hydrat umgewandelt ist.

8. Verfahren nach Anspruch 5 bis 7, wobei die in Schritt a) hergestellte Mischung mit Formulierungshilfsmitteln versetzt wird.

9. Verfahren nach Anspruch 5, **dadurch gekennzeichnet, dass** man die Lösung in Schritt a) von 30-70°C inkubiert.

10. Suspensionskonzentrat, enthaltend als wesentliche Komponente das Hydrat gemäß Anspruch 1 oder 2.

11. Mehrphasige wässrige Suspoemulsion enthaltend als wesentliche Komponente das Hydrat gemäß Anspruch 1 oder 2.

12. Verfahren zur Bekämpfung von phytopathogenen Pilzen, **dadurch gekennzeichnet, daß** man ein Suspensionskonzentrat nach Anspruch 10 oder eine mehrphasige, wässrige Suspoemulsion nach Anspruch 11 verdünnt und auf den jeweiligen Schädling oder die vor dem jeweiligen Schädling zu schützenden Materialen, Pflanzen, Boden und Saatgüter appliziert.

13. Verfahren nach Anspruch 12, **dadurch gekennzeichnet, dass** das Suspensionskonzentrat oder die Suspoemulsion jeweils mindestens einen weiteren fungiziden Wirkstoff enthalten.

14. Verfahren zur Bekämpfung von phytopathogenen Pilzen und gleichzeitiger Bekämpfung von unerwünschtem Insekten- oder Milbenbefall und/oder zur Regulation des Wachstums von Pflanzen, **dadurch gekennzeichnet, daß** man ein Suspensionskonzentrat nach Anspruch 10 oder eine mehrphasige, wässrige Suspoemulsion nach Anspruch 11 verdünnt und auf den jeweiligen Schädling oder die vor dem jeweiligen Schädling zu schützenden Materialen, Pflanzen, Boden und Saatgüter appliziert, wobei das Suspensionskonzentrat oder die Suspoemulsion jeweils mindestens einen weiteren agrochemischen Wirkstoff enthalten können.

## Claims

1. A crystalline hydrate of a substituted anilide derivative of the formula I 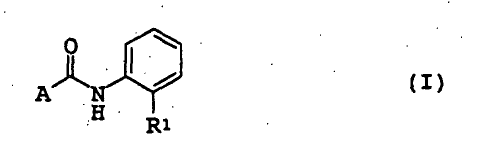 in which
A is 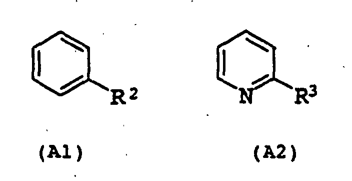
R¹ is phenyl which is substituted by halogen,
R² is methyl, difluoromethyl, trifluoromethyl, chlorine, bromine or iodine,
R³ is trifluoromethyl or chlorine.

2. A hydrate as claimed in claim 1 in the form of the monohydrate.

3. A method for the preparation of a hydrate as claimed in claim 1 or 2, comprising the following steps:
a) dissolving the anhydrate of I in a water-soluble organic solvent, and
b) precipitating the hydrate of I by addition of water.

4. A method as claimed in claim 3, wherein the organic solvent used is dioxane, tetrahydrofuran, dimethylformamide or N-methylpyrrolidone or mixtures thereof.

5. A method for the preparation of a hydrate as claimed in claim 1 or 2, comprising the following steps:
a) mixing an organic solution comprising the anhydrate of I with water,
b) heating the mixture prepared in step a) at a temperature of 30-150°C,
c) cooling the resulting solution.

6. A method as claimed in claim 5, wherein the organic solvent used is benzene, toluene, xylene, tetrahydrofuran, dimethylformamide or N-methylpyrrolidone or mixtures thereof.

7. A method for the preparation of a hydrate as claimed in claim 1 or 2, comprising the following steps:
a) mixing the solid anhydrate of I with water,
b) heating the mixture prepared in step a) at a temperature of 30-150°C until the anhydrate has been converted into the hydrate, or
c) incubating the mixture prepared in step a) with exposure to shearing forces until the anhydrate has been converted into the hydrate.

8. A method as claimed in any of claims 5 to 7, where the mixture prepared in step a) is mixed with formulation auxiliaries.

9. A method as claimed in claim 5, wherein the solution is incubated in step b) at 30-70°C.

10. A suspension concentrate comprising, as essential component, the hydrate as claimed in claim 1 or 2.

11. A multiphase suspoemulsion comprising, as essential component, the hydrate as claimed in claim 1 or 2.

12. A method of controlling phytopathogenic fungi, which comprises diluting a suspension concentrate as claimed in claim 10 or a multiphase aqueous suspoemulsion as claimed in claim 11 and applying the solution to the pest in question or to the materials, plants, soil and seeds to be protected from the pest in question.

13. A method as claimed in claim 12, wherein the suspension concentrate or the suspoemulsion comprises in each case at least one further fungicidal active ingredient.

14. A method for the control of phytopathogenic fungi and the simultaneous control of undesired attack by insects or mites and/or for regulating the growth of plants, which comprises diluting a suspension concentrate as claimed in claim 10 or a multiphase aqueous suspoemulsion as claimed in claim 11 and applying the dilute product to the pest in question or to the materials, plants, soil and seeds to be protected from the pest in question, it being possible for the suspension concentration or the suspoemulsion to comprise in each case at least one further agrochemical active ingredient.

## Revendications

1. Hydrates cristallins de dérivés d'anilide substitués de la formule I : 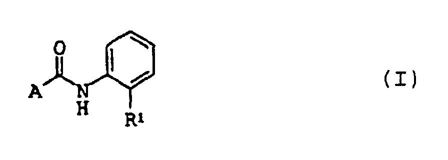 dans laquelle
A représente 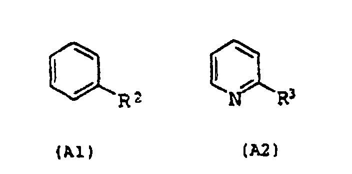
R¹ représente un groupe phényle qui est substitué par de l'halogène,
R² représente un groupe méthyle, difluorométhyle ou trifluorométhyle, du chlore, du brome ou de l'iode,
R³ représente un groupe trifluorométhyle ou du chlore.

2. Hydrates suivant la revendication 1, qui se présentent sous la forme de monohydrates.

3. Procédé de préparation d'hydrates suivant l'une des revendications 1 et 2, qui comprend les étapes suivantes :
a) une dissolution de l'anhydrate de formule I dans un solvant organique soluble dans l'eau, et
b) une précipitation de l'hydrate de formule I par addition d'eau.

4. Procédé suivant la revendication 3, **caractérisé en ce que**, comme solvant organique, on utilise du dioxanne, du tétrahydrofuranne, du diméthylformamide ou de la N-méthylpyrrolidone ou des mélanges des solvants précités.

5. Procédé de préparation d'hydrates suivant l'une des revendications 1 et 2, qui comprend les étapes suivantes :
a) un mélange d'une solution organique contenant l'anhydrate de formule I avec de l'eau,
b) un chauffage du mélange préparé dans l'étape a) à une température de 30-150°C,
c) un refroidissement de la solution préparée.

6. Procédé suivant la revendication 5, **caractérisé en ce que**, comme solvant organique, on utilise du benzène, du toluène, du xylène, du tétrahydrofuranne, du diméthylformamide ou de la N-méthylpyrrolidone ou des mélanges des solvants précités.

7. Procédé de préparation d'hydrates suivant l'une des revendications 1 et 2, qui comprend les étapes suivantes :
a) un mélange de l'anhydrate solide de formule I avec de l'eau,
b) un chauffage du mélange préparé dans l'étape a) à une température de 30-150°C jusqu'à ce que l'anhydrate soit transformé en l'hydrate, ou
c) une incubation du mélange préparé dans l'étape a), celui-ci étant exposé à des forces de cisaillement jusqu'à ce que l'anhydrate soit transformé en l'hydrate.

8. Procédé suivant l'une des revendications 5 à 7, dans lequel le mélange préparé dans l'étape a) est additionné d'adjuvants de formulation.

9. Procédé suivant la revendication 5, **caractérisé en ce qu'**on met la solution de l'étape a) à incuber à 30-70°C.

10. Concentré de suspension, contenant, comme composant essentiel, l'hydrate suivant l'une des revendications 1 et 2.

11. Suspoémulsion aqueuse à plusieurs phases, contenant, comme composant essentiel, l'hydrate suivant l'une des revendications 1 et 2.

12. Procédé pour lutter contre des champignons phyto-pathogènes, caractérisé en qu'on dilue un concentré de suspension suivant la revendication 10 ou une suspoémulsion aqueuse à plusieurs phases suivant la revendication 11 et en ce qu'on l'applique sur le parasite respectif ou sur les matières, plantes, sols et semences à protéger du parasite respectif.

13. Procédé suivant la revendication 12, **caractérisé en ce que** le concentré de suspension ou la suspoémulsion contiennent respectivement au moins une autre substance active fongicide.

14. Procédé pour lutter contre des champignons phyto-pathogènes et lutter simultanément contre une infestation non souhaitée par des insectes ou par des acariens et/ou pour réguler la croissance des plantes, **caractérisé en ce qu'**on dilue un concentré de suspension suivant la revendication 10 ou une suspoémulsion aqueuse à plusieurs phases suivant la revendication 11 et **en ce qu'**on l'applique sur le parasite respectif ou les matières, plantes, sols et semences à protéger du parasite respectif, le concentré de suspension ou la suspoémulsion pouvant respectivement contenir au moins une autre substance active agrochimique.
